(19) Europäisches Patentamt
European Patent Office
Office européen des brevets

(11) **EP 4 614 178 A1**

(12) **EUROPEAN PATENT APPLICATION**

(43) Date of publication:
**10.09.2025 Bulletin 2025/37**

(21) Application number: **24162241.4**

(22) Date of filing: **08.03.2024**

(51) International Patent Classification (IPC):
**G01R 33/565** (2006.01)   **A61B 5/055** (2006.01)
**G01R 33/48** (2006.01)

(52) Cooperative Patent Classification (CPC):
**G01R 33/56509;** A61B 5/055; G01R 33/4824;
G01R 33/4826

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.
5656 AG Eindhoven (NL)**

(72) Inventors:
• **NIELSEN, Tim**
  **Eindhoven (NL)**
• **LEE, Yoo Jin**
  **Eindhoven (NL)**

(74) Representative: **Philips Intellectual Property &
Standards
High Tech Campus 52
5656 AG Eindhoven (NL)**

(54) **MOTION CORRECTION USING NAVIGATORS BETWEEN SEQUENTIAL GROUPS OF K-SPACE DATA**

(57)    Disclosed herein is a method of medical imaging and an associated medical system. The method comprises: receiving (200) k-space data (114, 114'), wherein the k-space data comprises sequential groups (500, 502, 504), wherein the sequential groups comprise lines of k-space data, wherein adjacent pairs of the sequential groups comprise duplicated lines (510, 512, 514) of k-space data unique to each of the adjacent pairs; calculating (202) a motion detection metric (116) for the adjacent pairs of the sequential groups using the respective duplicated lines of k-space data; combining (204) adjacent pairs of the sequential groups if the respectively calculated motion detection metric is below a predetermined threshold to reform the sequential groups; and reconstructing (206) a motion corrected magnetic resonance image (122) from the reformed sequential groups (118) of k-space data using a joint optimization algorithm (120) that simultaneously provides the motion corrected magnetic resonance image and motion transformations between the reformed sequential groups, and wherein the motion corrected magnetic resonance image is at least partially reconstructed using the duplicated lines of k-space data.

Fig. 2

## Description

### TECHNICAL FIELD

The invention relates to magnetic resonance imaging, in particular to motion correction in magnetic resonance imaging.

### BACKGROUND

A large static magnetic field is used by Magnetic Resonance Imaging (MRI) scanners to align the nuclear spins of atoms as part of the procedure for producing images within the body of a patient. This large static magnetic field is referred to as the B0 field or the main magnetic field. During a magnetic resonance imaging scan or procedure, various magnetic field gradients and radiofrequency (RF) pulses are applied to the nuclear spins which then emit RF signals. The RF signals are sampled at various points and recorded. These samples are measurements made in k-space and are referred to as k-space data.

A challenge in performing magnetic resonance imaging is that the k-space data needed to reconstruct a magnetic resonance image can take minutes of time to acquire. If the subject moves this can result in artifacts in the magnetic resonance image.

Polak, D. et al, "Motion guidance lines for robust data consistency-based retrospective motion correction in 2D and 3D MRI," Magnetic Resonance in Medicine, 89 (2023), pp. 1777-1790, discloses a retrospective motion-correction technique based on repeating k-space guidance lines for improving motion correction in Cartesian 2D and 3D brain MRI. The motion guidance lines are inserted into the standard sequence orderings for 2D turbo spin echo and 3D MPRAGE to inform a data consistency-based motion estimation and reconstruction, which can be guided by a low-resolution scout. The extremely limited number of required guidance lines are repeated during each echo train and discarded in the final image reconstruction. Thus, integration within a standard k-space acquisition ordering ensures the expected image quality/contrast and motion sensitivity of that sequence.

### SUMMARY OF THE INVENTION

The invention provides a medical system, a method, and a computer program in the independent claims. Embodiments are given in the dependent claims.

In one aspect a method of medical imaging is disclosed. The method comprises receiving k-space data. The k-space data comprises sequential groups. The sequential groups comprise lines of k-space data. Adjacent pairs of the sequential groups comprise duplicate lines of k-space data unique to each of the adjacent pairs. The method further comprises calculating a motion detection metric for the adjacent pairs of the sequential groups using the respective duplicated lines of k-space data. The method further comprises combining adjacent pairs of the sequential groups if the respectively calculated motion detection metric is below a predetermined threshold to reform, or with other words regroup, the sequential group. The method further comprises reconstructing a motion corrected magnetic resonance image from the reformed sequential groups of k-space data using a joint optimization reconstruction algorithm that simultaneously provides the motion corrected magnetic resonance image and motion transformations between the sequential groups. The motion corrected magnetic resonance image is at least partially reconstructed using the duplicated lines of k-space data.

In another aspect a medical system is disclosed. The medical system comprises a computational system. The computational system is configured to receive k-space data. The k-space data comprises sequential groups. The sequential groups comprise lines of k-space data. Adjacent pairs of the sequential groups comprise duplicate lines of k-space data unique to each of the adjacent pairs. The computational system is further configured to calculate a motion detection metric for the adjacent pairs of the sequential groups using the respective duplicated lines of k-space data. The computational system is further configured to combine adjacent pairs of the sequential groups if the respectively calculated motion detection metric is below a predetermined threshold to reform the sequential groups. The computational system is further configured to reconstruct or generate a motion corrected magnetic resonance image from the reformed sequential groups of k-space data using a joint optimization reconstruction algorithm that simultaneously provides the motion corrected magnetic resonance image and motion transformations between the sequential groups. The motion corrected magnetic resonance image is at least partially reconstructed using the duplicated lines of k-space data.

In another aspect a computer program is disclosed. The computer program is configured for causing a computational system to perform a method of medical imaging.

The computer program may be present either on a data carrier or be present in a data network so as to be downloaded for installation in the control unit of the MR system. The processing of the acquired MR signals for the reconstruction of MR images may also be performed on a computer which is separate from the MR system used for acquisition of the MR signals.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the method and system, as defined in the claims, is provided in the following written description of various embodiments of the disclosure and illustrated in the accompanying drawings.

**BRIEF DESCRIPTION OF THE DRAWINGS**

In the following preferred embodiments of the invention will be described, by way of example only, and with reference to the drawings in which:

Fig. 1 illustrates an example of a medical system.
Fig. 2 shows a flow chart which illustrates a method according to the invention.
Fig. 3 illustrates a further example of a medical system.
Fig. 4 shows a flow chart which illustrates a further method according to the invention.
Fig. 5 illustrates two examples of k-space data divided into sequential groups.

**DESCRIPTION OF EMBODIMENTS**

For the purposes of promoting and understanding the principles of the present disclosure, reference will now be made to the embodiments illustrated in the drawings, and specific language will be used to describe the same. It is nevertheless understood that no limitation to the scope of the disclosure is intended. Any alteration and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately. Features described in relation to a method, may be implemented in a system, a computer implemented method and/or in a computer program product, in a corresponding manner.

In an example, a medical system comprises a memory storing machine-executable instructions. The medical system further comprises a computational system. The computational system may for example be one or more computational systems or computing cores which are located at one or more locations. The computational system may for example be implemented into a variety of situations. In one example the computational system may be implemented into the computer or controller for a magnetic resonance imaging system. In another example, the computational system may be a workstation used in a radiology or other medical setting. In yet another example the computational system may be accessible via the internet or other network connection and may for example be a cloud-based system which provides services for the reconstruction of magnetic resonance images.

Execution of the machine-executable instructions causes the computational system to receive k-space data. The k-space data comprises sequential groups. The sequential groups comprise lines of k-space data. The sequential groups may correspond to acquisitions of k-space data. When k-space data is acquired for reconstructing a magnetic resonance image it is typically acquired using multiple acquisitions. A difficulty with this is that this may take an extended amount of time. For example, between individual acquisitions which correspond to sequential groups the subject may move. The adjacent pairs of the sequential groups comprise duplicated lines of k-space data unique to each of the adjacent pairs.

A line of k-space (a line of k-space data) as used herein encompasses k-space data acquired after an RF excitation. A line of k-space data is therefore k-space data acquired along a trajectory in k-space. The groups of k-space data are formed from lines of k-space data that were acquired within a predetermined amount of time duration. By dividing the k-space data into sequential groups, the k-space data acquired at approximately the same time is binned together.

The duplicated lines of k-space data may be used as a navigator between the adjacent pairs. Typically, when navigator lines are added to an acquisition they are duplicated in every acquisition so that it is possible to determine if the subject has moved during the individual acquisitions which correspond to the sequential groups. A difficulty with this is that the data is repeatedly acquired over and over again which has the result of increasing the amount of time which it takes to acquire the k-space data. Instead of repeating the same lines of k-space data over and over again as a navigator, the duplicated lines function as a navigator only between these adjacent pairs. This means that there was less repetition of the k-space data used for navigators and this resulted in the acquisition time for the sequential groups to be reduced. Thie reduced scan time reduces the chance that the subject will move during the scan. This may lead to k-space data which contains reduced motion artifacts.

Execution of the machine-executable instructions further causes the computational system to calculate a motion detection metric for the adjacent pairs of the sequential groups using the respective duplicated lines of k-space data. The motion detection metric may for example be a single numerical value which is calculated by comparing the values in the duplicated lines of k-space data. For example, the difference between individual values could be calculated and then squared and then added together. If the motion detection metric is above a predetermined threshold this may mean that the subject moved between the acquisition of the adjacent pair.

Execution of the machine-executable instructions further causes the computational system to combine adjacent pairs of the sequential groups if the respectively calculated motion detection metric is below a predetermined threshold to reform the sequential groups. In this feature, if the motion detection metric for a particular adjacent pair of the sequential groups is below a predetermined threshold, then they are deemed to not have significant motion and are grouped together. This has significant benefits in accelerating the reconstruction of the magnetic resonance image.

There are several modalities to determine the threshold, from which the simplest and most general method is based on a statistical analysis of the distribution of the metric values of the whole set of adjacent pairs. Typically, the number of adjacent pairs is fairly large (>100) and patient motion is a rare event. The distribution will contain many values where no motion occurred between adjacent pairs. These values all cluster at the low end of the distribution of values because motion events always lead to an increase of the metric. The mean of the peak plus its width multiplied by a quality factor can be used as threshold for the combination.

This quality factor can be used to balance the probability of making a wrong decision (i.e. combining groups although there was motion) against being too conservative and gaining no speedup because the combination threshold is set too low.

Setting the quality factor can be left to the operators of the system to express their preference (reconstruction speed vs. image quality). For example, it could be made a user interface parameter, which can be set either by introducing a value, selecting from a menu comprising a set of displayed values, selecting by a sliding graded scale. There is no risk for the patient because one can always perform another reconstruction with a more conservative setting if the first reconstruction comes out with relatively poor quality. Additionally or alternatively, the quality factor in some of the examples may be set depending on sequence and anatomy.

In some embodiments use of an internal table, e.g., set of values in a displayed menu, that is the result of typical results obtained from clinical testing may be effected. The result may be averaged over many patients. It is an experimental finding, that the distribution of values of the motion detection metric in the absence of motion is patient specific (most likely due to micro-motion like blood flow). Alternatively, in some embodiments instead of analyzing the width of the peak at the low end of the distribution, one can also use more elaborated methods, e.g., by training an artificial intelligence module that obtains the metric values of all adjacent pairs as input and which outputs the combined groups.

Execution of the machine-executable instructions further causes the computational system to reconstruct a motion corrected magnetic resonance image from the reformed sequential groups of k-space data using a joint optimization reconstruction algorithm that simultaneously provides the motion corrected magnetic resonance image and motion transformation between the sequential groups. The motion corrected magnetic resonance image is at least partially reconstructed using the duplicated lines of k-space data. The joint optimization is a known method of performing motion correction for k-space data when it has been divided into groups. Examples may have the benefit of accelerating the joint optimization by combining the adjacent pairs of the sequential groups when it is detected that there is not significant motion between the groups. This may result in huge numerical savings during the reconstruction.

In another example, execution of the machine-executable instructions further causes the computational system to calculate initial rigid body motion parameters using the respective duplicate lines of k-space data of the adjacent pairs of the sequential groups. The duplicated lines that are shared between an adjacent pair of the sequential groups may be used to determine the relative motion between those two particular groups. As such, these can be used as a means to determine the motion state of a particular group with respect to a reference or to the initial sequential group. Execution of the machine-executable instructions further causes the computational system to initialize the joint optimization using the initial rigid body motion parameters.

In a joint optimization the motion parameters are determined by solving an optimization problem. Determining the proper location to properly solve this can be computationally intensive or there is always the risk of finding a local minimum as the solution. By using the initial rigid body motion parameters, the joint optimization can be started near to the proper solution. This may have the advantage of not only reducing the computational overhead of solving the joint optimization but also to ensure that the proper solution is found.

An exemplary joint optimization for estimating the motion-free image (x) and motion parameters ($\theta$) can be formulated as follows:

$$\left(\hat{\mathbf{x}}, \widehat{\boldsymbol{\theta}}\right) = \underset{\mathbf{x},\boldsymbol{\theta}}{\mathrm{argmin}} \|\mathbf{AFST}_{\boldsymbol{\theta}}\mathbf{x} - \boldsymbol{y}\|_2^2 \qquad \text{Eq. (1)}$$

where T is a set of parameterized motion transformations, S are the coil sensitivities, F is the discrete Fourier transform, and A is a sampling mask. Here, a fixed number of motion states during the data acquisition (M) is assumed. The joint optimization alternates between the image reconstruction with the current estimate of motion parameters (θ) and motion update with the current estimate of motion-free image (**x**). When updating motion parameters of each segment, only a small portion of k-space data corresponding to one motion segment is used. Thus, the motion estimation could suffer if the number of motion segments and/or the native under-sampling factor of the scan is too high.

Examples may acquire additional navigator lines for more robust motion estimation. Rather than acquiring navigator lines repeatedly at the same k-space position as in the traditional approach, here the k-space positions of the navigator lines are determined for each shot such that k-space gaps are more effectively filled up while providing sufficient information about motion. For the estimation of relative motion between shots, half of the navigator lines may overlap with the preceding shot and the other half in one example.

In another example, the joint optimization comprises minimizing an objective function depending on the motion corrected magnetic resonance image and the motion transformations between the sequential groups. The objective function is calculated as a loss function applied to a difference between the measured k-space data and k-space data calculated from the motion corrected magnetic resonance image modified by the motion parameters. This may have the advantage of providing an accurate means of determining the motion correction for the entire group of k-space data.

In another example, the loss function is the motion detection metric. The use of the loss function as being the same as the motion detection metric has the advantage that when adjacent pairs of the sequential groups are combined, it is then known what would be an acceptable or normal value of the loss function when the motion has been corrected properly. This may for example be used in setting the conditions for upping the joint optimization. This may have the benefit of reducing the computational overhead.

In another example, the motion detection metric is an L2 norm calculated from the difference of the duplicated lines of k-space data. This example may have the advantage that it is computationally easy to determine and yields accurate results. There are many modifications conceivable, for example introducing weighting factors for the simple L2 norm, that take the k-space position of the duplicated lines into account.

Other example embodiments may use another form of motion detection metric that can be generated by machine learning. Input to the metric are the measured data of the duplicated k-space lines, output is a single value, e.g., number, that indicates motion, which can for example be the probability for motion, or the estimated displacement. This is a typical regression problem for which many classical machine-learning and neural-network based techniques are known. All of these methods need training data in the form of pairs of input and output values. For the purpose of learning a motion detection metric, these pairs of data can be created by using (motion artifact free) MRI images of the sequence of interest and simulate k-space data with and without applying a motion transform to the image volume (and adding the appropriate noise) - these forming the input data. The displacement of the motion transform used in the simulation is the target output value of the motion detection metric. This simulation can be repeated with a range of motion parameters (and of course also without or with negligible motion) and with MRI images of different subjects to generate a suitably large training data set.

In another example, the method further comprises calculating the average motion detection metric for the combined adjacent pairs. The method further comprises halting the joint optimization reconstruction algorithm to provide the motion corrected magnetic resonance image if the loss function is less than or equal to the average motion detection metric times a predetermined constant. This example illustrates that when the loss function and the motion detection metric are the same it provides a computational benefit.

In another example, the motion transformations between the sequential groups consist of sets of predetermined motion states. For example, instead of allowing a continuous variable, the certain discrete values of a rotation or transformation may only be allowed. This has the benefit of accelerating the solution of the joint optimization.

In another example, the duplicated lines of k-space data unique to each of the adjacent pairs has the property of the duplicate lines being located within a predetermined maximum distance and predetermined minimum distance from a center of k-space. This example may have the benefit that being near the center of k-space these points may function better as a navigator.

In another example, the duplicated lines of k-space data unique to each of the adjacent pairs has the property of the duplicated lines within a single sequential group of the sequential groups are separated by at least a predetermined minimum distance in k-space. Having them separated within k-space may result in them being more effective as navigators and also ensuring that they are more useful or contribute more to a reconstruction of the magnetic resonance image.

In another example, the duplicated lines of k-space data unique to each of the adjacent pairs has a property of the duplicated lines within the single sequential group are located within regions of k-space data where a k-space line density is below a predetermined k-space line density. The duplicated lines are then placed where less k-space data is being acquired. This means that they may make a larger contribution to the reconstructed image.

In another example, the medical system further comprises a magnetic resonance imaging system. The memory further stores pulse sequence commands configured for acquiring the sequential groups of the k-space data sequentially. Execution of the machine-executable instructions further causes the computational system to acquire the k-space data by controlling the magnetic resonance imaging system with the pulse sequence commands.

In another example the pulse sequence commands are configured to sample the k-space data according to a Distributed and Incoherent Sample Orders for Reconstruction Deblurring using Encoding Redundancy (DISORDER) method. The motion corrected magnetic resonance image could be reconstructed according to the DISORDER method or protocol also. In this case a joint optimization, such as is presented in Eq. 1 above could be used for the reconstruction. Motion is one of the main sources of artifacts in MRI that degrade diagnostic utility, especially for long, high-resolution 3D scans. It often requires repeated scans, resulting in increased costs. The retrospective motion compensation approaches DISORDER corrects motion artifacts after data acquisition, by incorporating the effect of motion into the encoding matrix and by jointly estimating a motion-free image and motion parameters. This approach solely relies on encoding redundancy in the measured data to estimate motion by modifying k-space sample orders appropriately. This data-driven approach is attractive because it is readily applicable in clinical examinations without the need for extra hardware or sequence modifications. However, extracting motion information from a segment of k-space data becomes difficult as the number of motion segments increases, which could result in images with remaining motion artifacts.

Even for a relatively lower number of motion segments, the motion estimation could be challenging for the scans acquired with high under-sampling factors.

Navigator echoes have been shown to be an important method for tracking motion in MRI. It is acquired repeatedly without phase encoding (which corresponds to the k-space line at ky=0 for 2D scans and (ky,kz)=(0,0) for 3D scans), providing information about motion along readout direction. Alternatively, a 3D spherical navigator could be used to estimate rigid body motion in all three directions. It is possible for repeated acquisition of k-space guidance lines (2 or 4 guidance lines for 2D or 3D scans, respectively) (a guidance line is duplicated lines of k-space unique to each of the adjacent pairs of the sequential groups) that have a fixed distance from the k-space center during each echo train to enable motion estimation and correction. These navigator data (or guidance lines / duplicated lines of k-space data) are excluded from the image reconstruction, as they are acquired at the same k-space position repeatedly.

Examples may provide a new way of acquiring navigator data for the DISORDER approach that can be used for the motion estimation and image reconstruction. One common argument against using navigator echoes is that they require additional scan-time. In this example this is not the case, because the navigator data are used in the reconstruction just as the other data. The introduction of navigators merely changes the sampling density by acquiring selected lines in more than one shot.

In another example, the k-space data is acquired according to a magnetic resonance imaging protocol. The magnetic resonance imaging protocol is any one of the following: a magnetization-prepared rapid acquisition gradient echo (MP-RAGE), a fast spin echo (FSE), a fluid attenuated inversion recovery (FLAIR), a spoiled gradient echo (SPGR), and a balanced steady-state free procession (bSSFP).

In another example, execution of the machine-executable instructions further causes the computational system to perform the calculation of the motion detection metric for the adjacent pairs of the sequential groups using the duplicated lines of k-space data during the acquisition of the k-space data by the magnetic resonance imaging system. Execution of the machine-executable instructions further causes the computational system to perform any one of the following if the motion detection metric is above a predetermined warning value: halt acquisition of the k-space data, trigger a reacquisition of the k-space data, provide a subject motion warning signal, and combinations thereof. This example may be beneficial because it may provide for a means of repeating measurements instead of just performing retrospective motion correction.

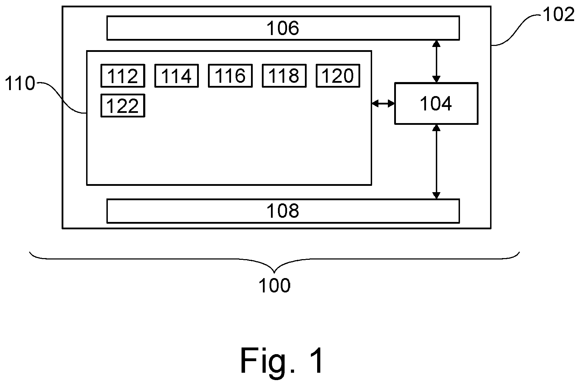

Fig. 1 illustrates an example of a medical system 100. The medical system 100 is shown as comprising a computer 102. The computer 102 may represent one or more computers located at one or more locations. The computer 102 could for example be different types of computers. It could be a computer 102 used to control and operate a magnetic resonance imaging system, it could be a workstation used by a radiologist or other medical professionals, and it could also be a remote server or virtual computing system that provides image reconstruction services on demand. The computer 102 is shown as comprising a computational system 104. The computational system 104 represents one or more computational systems or computer cores located at one or more locations.

The computational system 104 is shown as being in communication with an optional hardware interface 106, an optional user interface 108, and a memory 110. The hardware interface 106 may for example provide network conductivity to the computer 102 as well as enable the computational system 104 to exchange data with and to control other components of the medical system 100 if they are present. For example, the hardware interface 106 may enable the operation and control of a magnetic resonance imaging system. The user interface 108 may provide a means for an operator or user to operate and control the operation and function of the medical system 100. The memory 110 is intended to represent various types of memory 110 which may be accessible to the computational system 104. In some examples the memory 110 at least partially comprises a non-transitory storage medium.

The memory 110 is shown as containing machine-executable instructions 112. The machine-executable instructions 112 provide instructions which enable the computational system 104 to provide numerical and image processing tasks as well as control other components via the hardware interface 106. The memory 110 is further shown as containing k-space data 114 that is divided into sequential groups. The k-space data contains lines of k-space data. The adjacent pairs of the sequential groups comprise duplicate lines of k-space data that are unique to each of the adjacent pairs. The memory 110 is further shown as containing motion detection metric values 116 that were calculated between adjacent pairs of the sequential groups. The memory 110 is further shown as containing k-space data divided into reformed groups 118. The motion detection metric values 116 were used to detect if there was motion between when the various sequential groups were acquired. If a pair of the sequential groups was shown as having the same or close enough motion state then they would be binned or reformed into a larger group. The memory 110 is further shown as containing a joint optimization algorithm 120. The memory 110 is further shown as containing a motion corrected magnetic resonance image 122 that was reconstructed by applying the joint optimization algorithm 120 to the k-space data divided into the reformed groups 118.

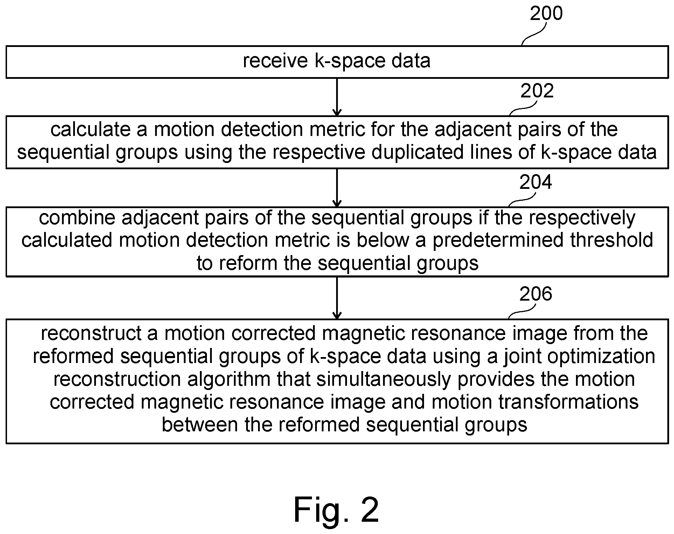

Fig. 2 is a flowchart that illustrates a method, which may be used for operating the medical system 100 of Fig. 1. In step 200, the k-space data 114 is received. The k-space data comprises sequential groups. They may for example have been acquired sequentially using a magnetic resonance imaging system. The sequential groups 114 also comprise individual lines of k-space data. A line, as used herein, encompasses a trajectory where data is acquired in k-space. The adjacent pairs of the sequential groups comprise duplicated lines of k-space data that are unique to each of the adjacent pairs. Next, in step 202, a motion detection metric 116 is calculated for the adjacent pairs of the sequential groups using the respective duplicated lines of k-space data. In step 204, the adjacent pairs of the sequential groups are combined if the respectively calculated motion detection metric is below a predetermined threshold in order to reform, or with other words regroup, the sequential groups into a smaller number of sequential groups. In step 206, a motion corrected magnetic resonance image 122 is reconstructed or generated from the reformed sequential groups of k-space data 118 using the joint optimization algorithm 120.

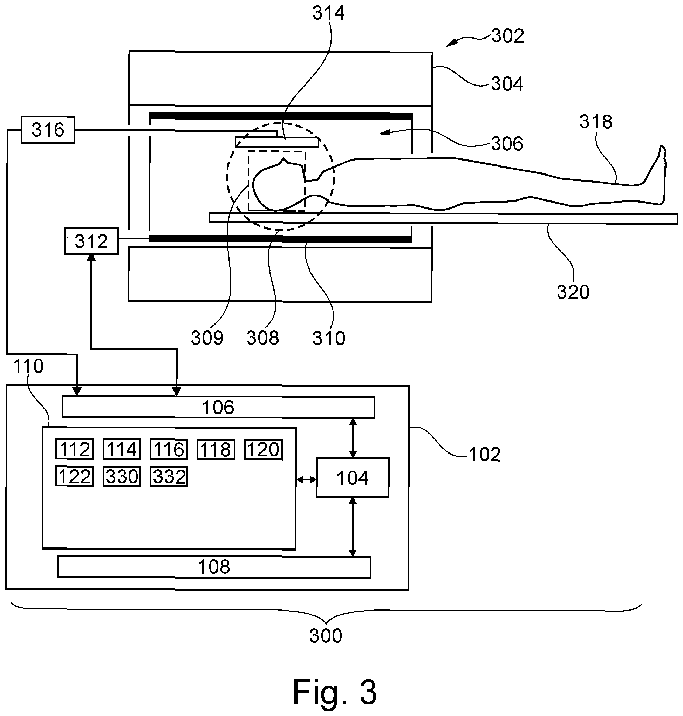

Fig. 3 illustrates a further example of a medical system 300. The medical system 300 depicted in Fig. 3 is similar to the medical system 100 depicted in Fig. 1 except that it additionally comprises a magnetic resonance imaging system 302. The magnetic resonance imaging system 302 comprises a magnet 304. The magnet 304 is a superconducting cylindrical type magnet with a bore 306 through it. It is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject. The arrangement of the two sections area is similar to that of a Helmholtz coil. Open magnets are popular because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A field of view 309 is shown within the imaging zone 308. The measured k-space data is acquired for the field of view 309. The region of interest could be identical with the field of view 309 or it could be a sub volume of the field of view 309. A subject 318 is shown as being supported by a subject support 320 such that at least a portion of the subject 318 is within the imaging zone 308 and the field of view 309. In this example, the head of the subject 318 is positioned within the field of view 309. The measured k-space data that will be acquired is therefore for performing a head or brain scan.

Within the bore 306 of the magnet there is also a set of magnetic field gradient coils 310 which is used for the acquisition of measured k-space data to spatially encode magnetic spins within the imaging zone 308 of the magnet 304. The magnetic field gradient coils 310 are connected to a magnetic field gradient coil power supply 312. The magnetic field gradient coils 310 are intended to be representative. Typically, magnetic field gradient coils 310 contain three separate sets of coils for spatial encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio frequency coil 314 is connected to a radio frequency transceiver 316. The radio frequency coil 314 and radio frequency transceiver 316 may be replaced by separate transmit and receive coils and a separate transmitter and receiver. It is understood that the radio frequency coil 314 and the radio frequency transceiver 316 are representative. The radio frequency coil 314 is intended to also represent a dedicated transmit antenna and a dedicated receive antenna. Likewise, the transceiver 316 may also represent a separate transmitter and receiver. The radio frequency coil 314 may also have multiple receive/transmit elements and the radio frequency transceiver 316 may have multiple receive/transmit channels. The transceiver 316 and the gradient controller 312 are shown as being connected to the hardware interface 106 of the computer system 102.

The memory 110 is further shown as containing pulse sequence commands 330 which are configured to control the magnetic resonance imaging system 302 to acquire the k-space data 114 that has been divided into sequential groups. The memory 110 is further shown as containing a set of initial rigid body motion parameters 332 for the k-space data divided into reformed groups 118. These are essentially rough or approximate motion translations which can be used for initializing each of the reformed groups in the joint optimization algorithm 120.

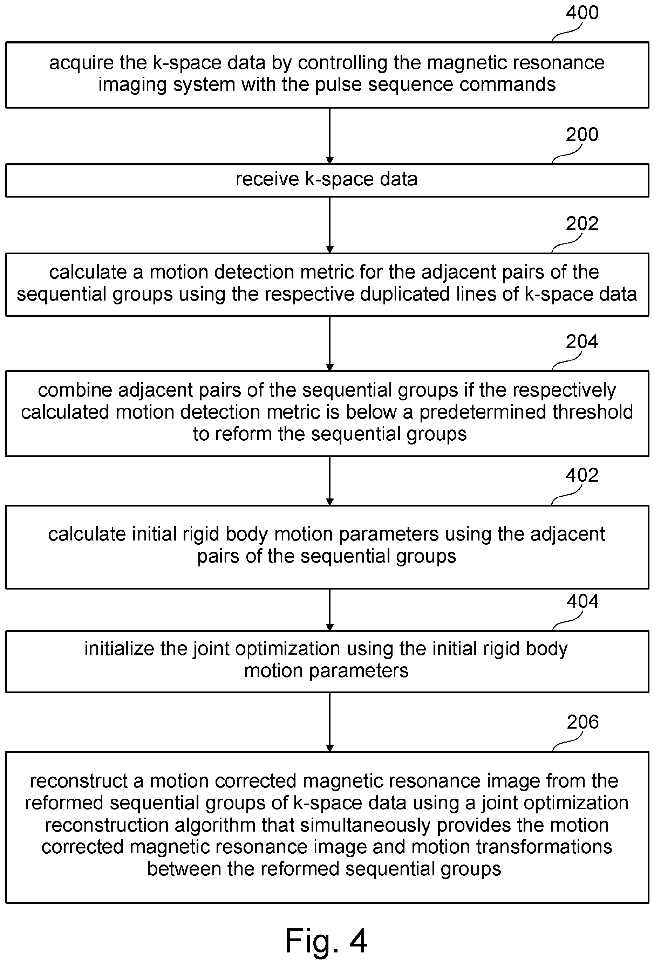

Fig. 4 shows a flowchart that illustrates a method that can be used in conjunction with the medical system 300 of Fig. 3. In step 400, the k-space data 114 is acquired by controlling the magnetic resonance imaging system 302 with the pulse sequence commands 330. Steps 200, 202, and 204 are performed as was illustrated in Fig. 2. In step 402, the initial rigid body motion parameters 332 are calculated using the k-space data that is divided into the reformed groups 118. The initial rigid body motion parameters are calculated by using the adjacent pairs of respective duplicated lines of k-space data. These provide the navigators which can be used to provide a rough estimate of a translation which transforms from one sequential group to the next. In step 404, the joint optimization algorithm 120 is initialized using the initial rigid body motion parameters 332. Step 206 is then performed as was illustrated in Fig. 2.

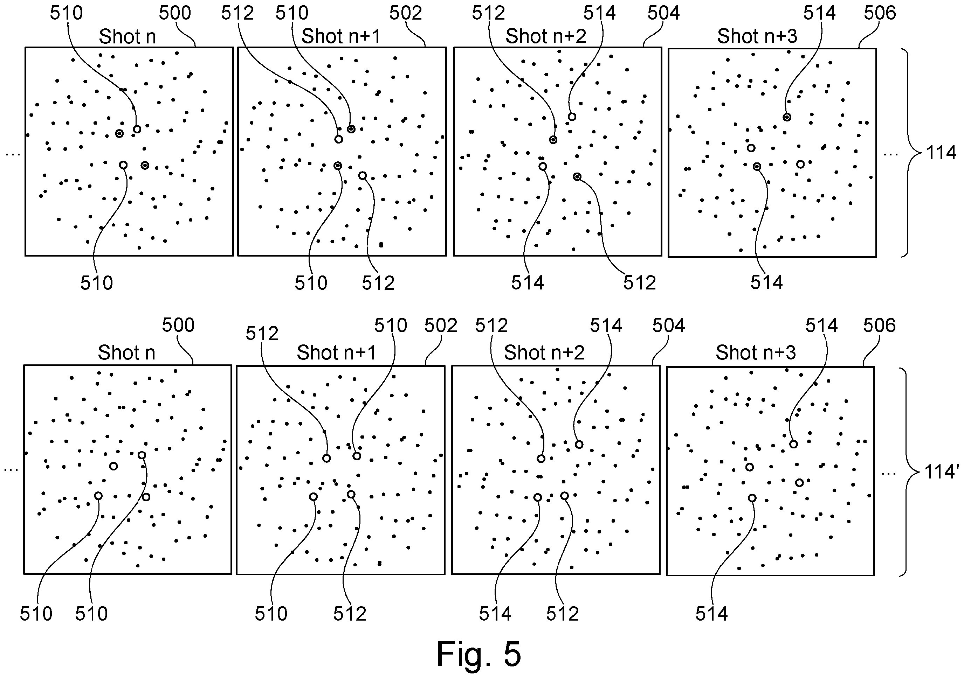

Fig. 5 illustrates two examples of k-space data 114, 114'. Both examples 114, 114' are shown as comprising sequential groups 500, 502, 504, 506 acquired during separate shots by the magnetic resonance imaging system 302. 500 is a group of k-space data corresponding to shot N. 502 is a group of k-space data corresponding to shot N+1. 504 is a group of k-space data corresponding to shot N+2. 506 is a group of k-space data corresponding to shot N+3. Shots N 500 and shot N+1 502 are an adjacent pair of the sequential groups. Shot N+1 502 and shot N+2 504 are also an adjacent pair. Shot N+2 504 and shot N+3 506 are also an adjacent pair of the sequential groups. The points in each of these shots, 500, 502, 504, and 506 correspond to k-space lines acquired during that shot in the DISORDER approach. The hollow circles 510, 512, 514 correspond to lines of k-space data which are used as navigators. These are the duplicated lines 510, 512, 514. The lines 510 are both replicated in shots N 500 and N+1 502. The lines 512 are replicated in shots N+1 502 and shot N+2 504. The lines 514 are replicated in shots N+2 504 and shot N+3 506. In the example 114, when there is a dot in the middle of the circle this means that the lines for the navigator were chosen from the original k-space lines in one of the shots. In the example 114' the circles 510, 512, 514 are all hollow and constitute additional lines which were added to the pulse sequence. Since all of these are additional this may contribute to additional scan time. However, in the example 114' the k-space positions were chosen to provide more flexibility for the navigator k-space positions.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

REFERENCE SIGNS LIST

| | |
|---|---|
| 100 | medical system |
| 102 | computer |
| 104 | computational system |
| 106 | optional hardware interface |
| 108 | optional user interface |
| 110 | memory |
| 112 | machine executable instructions |
| 114 | k-space data divided into sequential groups |
| 114' | k-space data divided into sequential groups |
| 116 | motion detection metric values |
| 118 | k-space data divided into reformed groups |
| 120 | joint optimization algorithm |
| 122 | motion corrected magnetic resonance image |
| 200 | receive k-space data |
| 202 | calculate a motion detection metric for the adjacent pairs of the sequential groups using the respective duplicated lines of k-space data |
| 204 | combine adjacent pairs of the sequential groups if the respectively calculated motion detection metric is below a predetermined threshold to reform the sequential groups |
| 206 | reconstruct a motion corrected magnetic resonance image from the reformed sequential groups of k-space data using a joint optimization reconstruction algorithm that simultaneously provides the motion corrected magnetic resonance image and motion transformations between the reformed sequential groups |
| 300 | medical system |
| 302 | magnetic resonance imaging system |
| 304 | magnet |
| 306 | bore of magnet |
| 308 | imaging zone |
| 309 | field of view |
| 310 | magnetic field gradient coils |
| 312 | magnetic field gradient coil power supply |

314     radio frequency coil
316     transceiver
318     subject
320     subject support
330     pulse sequence commands
332     initial rigid body motion parameters
400     acquire the k-space data by controlling the magnetic resonance imaging system with the pulse sequence command
402     calculate the initial rigid body motion parameters using the respective duplicate lines of k-space of the adjacent pairs of the sequential groups
404     initialize the joint optimization using the initial rigid body motion parameters
500     group of k-space data corresponding to shot n
502     group of k-space data corresponding to shot n + 1
504     group of k-space data corresponding to shot n + 2
506     group of k-space data corresponding to shot n + 3
510     duplicated lines for shot n and n+1
512     duplicated lines for shot n+1 and n+2
514     duplicated lines for shot n+2 and n+3

**Claims**

1. A medical system (100, 300) comprising a computational system configured to:

   receive (200) k-space data, wherein the k-space data (114, 114') comprises sequential groups (500, 502, 504), wherein the sequential groups comprise lines of k-space data, wherein adjacent pairs of the sequential groups comprise duplicated lines (510, 512, 514) of k-space data unique to each of the adjacent pairs;
   calculate (202) a motion detection metric (116) for the adjacent pairs of the sequential groups using the respective duplicated lines of k-space data;
   combine (204) adjacent pairs of the sequential groups if the respectively calculated motion detection metric is below a predetermined threshold to reform the sequential groups; and
   generate (206) a motion corrected magnetic resonance image (122) from the reformed sequential groups (118) of k-space data using a joint optimization algorithm (120) that simultaneously provides the motion corrected magnetic resonance image and motion transformations between the sequential groups, and wherein the motion corrected magnetic resonance image is at least partially generated using the duplicated lines of k-space data.

2. The medical system of claim 1, wherein the computational system is further configured to:

   calculate (402) initial rigid body motion parameters (332) using the respective duplicate lines of k-space of the adjacent pairs of the sequential groups; and
   initialize (404) the joint optimization algorithm using the initial rigid body motion parameters.

3. The medical system of claim 2, wherein the joint optimization algorithm comprises minimizing an objective function depending on the motion corrected magnetic resonance image and the motion transformations between the sequential groups, wherein the objective function is calculated as a loss function applied to a difference between the measured k-space data and k-space data calculated from the motion corrected magnetic resonance image modified by the motion parameters.

4. The medical system of claim 3, wherein the loss function is the motion detection metric.

5. The medical system of any of the preceding claims, wherein the motion detection metric is an L2 norm calculated from the difference of the duplicated lines of k-space data.

6. The medical system of claim 4 or 5, wherein the computational system is further configured to:

   calculate an average motion detection metric for combined adjacent pairs; and
   halt the joint optimization algorithm to provide the motion corrected magnetic resonance image if the loss function is less than or equal to the average motion detection metric times a predetermined constant.

7. The medical system of any one of the preceding claims, wherein the motion transformations between the sequential groups consist of a set of predetermined motion states.

8. The medical system of any one of the preceding claims, wherein the duplicated lines of k-space data unique to each of the adjacent pairs has any one of the following properties:

the duplicate lines are located within a predetermined maximum distance and predetermined minimum distance from a center of k-space;
the duplicated lines within a single sequential group of the sequential groups are separated by at least a predetermined minimum distance in k-space;
the duplicated lines within with the signal sequential group are located within regions of k-space with a k-space line density below a predetermined k-space line density; and
combinations thereof.

9. The medical system of any one of the preceding claims, wherein the medical system further comprises a magnetic resonance imaging system (302) and wherein the computational system is configured to acquire (400) the k-space data by controlling the magnetic resonance imaging system based on pulse sequence commands (330).

10. The medical system of claim 9, wherein the pulse sequence commands comprise any one of a magnetic resonance imaging protocol of: a magnetization-prepared rapid acquisition gradient echo, MP-RAGE, a fast spin echo, FSE, a fluid attenuated inversion recovery, FLAIR,, a spoiled gradient echo, SPGR, and a balanced steady-state free precession bSSFP.

11. The medical system of claim 9 or 10, wherein the computational system is further configured to:

perform the calculation of the motion detection metric for the adjacent pairs of the sequential groups using the duplicated lines of k-space data during the acquisition of the k-space data by the magnetic resonance imaging system; and
perform any one of the following if the motion detection metric is above a predetermined warning value: halt acquisition of the k-space data, trigger a reacquisition of the k-space data, provide a subject motion warning signal, and combinations thereof.

12. A method of medical imaging, comprising:

receiving (200) k-space data (114, 114'), wherein the k-space data comprises sequential groups (500, 502, 504), wherein the sequential groups comprise lines of k-space data, wherein adjacent pairs of the sequential groups comprise duplicated lines (510, 512, 514) of k-space data unique to each of the adjacent pairs;
calculating (202) a motion detection metric (116) for the adjacent pairs of the sequential groups using the respective duplicated lines of k-space data;
combining (204) adjacent pairs of the sequential groups if the respectively calculated motion detection metric is below a predetermined threshold to reform the sequential groups; and
generating (206) a motion corrected magnetic resonance image (122) from the reformed sequential groups (118) of k-space data using a joint optimization algorithm (120) that simultaneously provides the motion corrected magnetic resonance image and motion transformations between the reformed sequential groups, and wherein the motion corrected magnetic resonance image is at least partially generated using the duplicated lines of k-space data.

13. The method of claim 12, further comprising:

calculating (402) initial rigid body motion parameters using the adjacent pairs of the sequential groups; and
initializing (404) the joint optimization algorithm using the initial rigid body motion parameters.

14. The method of claim 13, wherein the joint optimization algorithm comprises minimizing an objective function depending on the motion corrected magnetic resonance image and the motion transformations between the sequential groups, wherein the objective function is calculated as a loss function applied to a difference between the measured k-space data and k-space data calculated from the motion corrected magnetic resonance image modified by the motion parameters.

15. A computer program comprising machine executable instructions, wherein execution of the machine executable instructions causes a computational system to perform a method according to any of the claims 12 to 14.

Fig. 1

200

receive k-space data

202

calculate a motion detection metric for the adjacent pairs of the sequential groups using the respective duplicated lines of k-space data

204

combine adjacent pairs of the sequential groups if the respectively calculated motion detection metric is below a predetermined threshold to reform the sequential groups

206

reconstruct a motion corrected magnetic resonance image from the reformed sequential groups of k-space data using a joint optimization reconstruction algorithm that simultaneously provides the motion corrected magnetic resonance image and motion transformations between the reformed sequential groups

Fig. 2

Fig. 3

400

acquire the k-space data by controlling the magnetic resonance imaging system with the pulse sequence commands

200

receive k-space data

202

calculate a motion detection metric for the adjacent pairs of the sequential groups using the respective duplicated lines of k-space data

204

combine adjacent pairs of the sequential groups if the respectively calculated motion detection metric is below a predetermined threshold to reform the sequential groups

402

calculate initial rigid body motion parameters using the adjacent pairs of the sequential groups

404

initialize the joint optimization using the initial rigid body motion parameters

206

reconstruct a motion corrected magnetic resonance image from the reformed sequential groups of k-space data using a joint optimization reconstruction algorithm that simultaneously provides the motion corrected magnetic resonance image and motion transformations between the reformed sequential groups

Fig. 4

Fig. 5

EP 4 614 178 A1

Europäisches
Patentamt

European
Patent Office

Office européen
des brevets

**EUROPEAN SEARCH REPORT**

Application Number

EP 24 16 2241

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2023/160989 A1 (POLAK DANIEL [DE] ET AL) 25 May 2023 (2023-05-25) * paragraph [0008] - paragraph [0022] * * paragraph [0029] * | 1-15 | INV. G01R33/565 A61B5/055 |
| A,D | POLAK DANIEL ET AL: "Motion guidance lines for robust data consistency-based retrospective motion correction in 2D and 3D MRI", MAGNETIC RESONANCE IN MEDICINE, vol. 89, no. 5, 6 February 2023 (2023-02-06), pages 1777-1790, XP093047025, US ISSN: 0740-3194, DOI: 10.1002/mrm.29534 Retrieved from the Internet: URL:https://onlinelibrary.wiley.com/doi/full-xml/10.1002/mrm.29534> * sections 1, 2 and 4; figures 2,4 * | 1-15 | ADD. G01R33/48 |
| A | US 2023/293039 A1 (POLAK DANIEL [DE] ET AL) 21 September 2023 (2023-09-21) * paragraph [0011] - paragraph [0024] * * paragraph [0033]; claims 1,2 * | 1-15 | TECHNICAL FIELDS SEARCHED (IPC) G01R |
| E | EP 4 386 415 A1 (SIEMENS HEALTHINEERS AG [DE]; MASSACHUSETTS GEN HOSPITAL [US]) 19 June 2024 (2024-06-19) * whole document, in particular paragraphs [0033], [0046-0050] and claims 1-7 and 13. * | 1-15 | |

The present search report has been drawn up for all claims

| Place of search | Date of completion of the search | Examiner |
|---|---|---|
| Munich | 5 August 2024 | Lebar, Andrija |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
..........................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 16 2241

This annex lists the patent family members relating to the patent documents cited in the above-mentioned European search report.
The members are as contained in the European Patent Office EDP file on
The European Patent Office is in no way liable for these particulars which are merely given for the purpose of information.

05-08-2024

| Patent document cited in search report | Publication date | Patent family member(s) | | Publication date |
|---|---|---|---|---|
| US 2023160989 A1 | 25-05-2023 | CN | 116165588 A | 26-05-2023 |
| | | EP | 4187271 A1 | 31-05-2023 |
| | | US | 2023160989 A1 | 25-05-2023 |
| US 2023293039 A1 | 21-09-2023 | EP | 4246170 A1 | 20-09-2023 |
| | | US | 2023293039 A1 | 21-09-2023 |
| EP 4386415 A1 | 19-06-2024 | EP | 4386415 A1 | 19-06-2024 |
| | | US | 2024201297 A1 | 20-06-2024 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **POLAK, D. et al.** Motion guidance lines for robust data consistency-based retrospective motion correction in 2D and 3D MRI. *Magnetic Resonance in Medicine*, 2023, vol. 89, 1777-1790